# EUROPÄISCHE PATENTANMELDUNG

(11) **EP 1 574 508 A1**
(43) Veröffentlichungstag der Anmeldung: **14.09.2005**
(21) Anmeldenummer: 04002055.4
(22) Anmeldetag: 30.01.2004
(51) Int. Cl.: C07D 317/44, C07D 491/08, C07D 209/52

(54) **Verfahren zur Herstellung von Acetalen und Ketalen von 3-Amino-5-(hydroxymethyl)-cyclopentan-1,2-diolen, sowie deren Derivaten und Salzen**

(71) Anmelder: Lonza AG, 4052 Basel (CH)
(72) Erfinder: Griffiths, Gareth-John, CH-3930 Visp (CH); Lange, Silvia, 3900 Brig (CH); Brieden, Walter, 3938 Ausserberg (CH)

(57) **Zusammenfassung**

Gegenstand der Erfindung ist ein Verfahren zur Herstellung von Acetalen und Ketalen von 3-Amino-5-(hydroxymethyl)cyclopentan-1,2-diolen der Formel 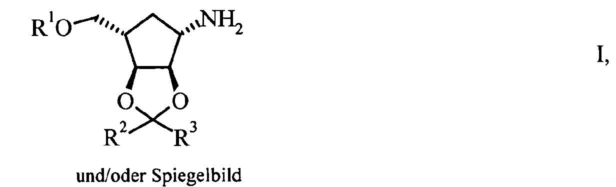 worin R¹ Wasserstoff, C₁₋₆-Alkyl, C₃₋₈-Cycloalkyl oder Benzyl sowie R² und R³ eine Kombination aus i) jeweils unabhängig voneinander Methyl und Ethyl ii) Wasserstoff und C₁₋₆-Alkyl oder Phenyl oder iii) eine Gruppe der Formel -(CH₂)ₙ- mit n = 4 bis 6 bedeuten, und die als freie Amine oder als Salze zwei- oder dreibasiger organischer Säuren vorliegen, ausgehend von 2-Acetyl-2-azabicyclo[2.2.1]hept-5-en-3-on der Formel

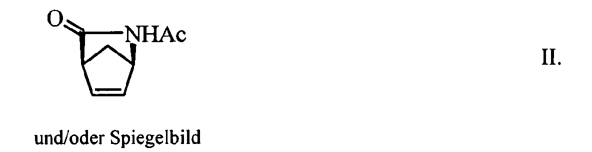

Das Verfahren ist, abhängig von der Zusammensetzung des Eduktes, gleichermassen geeignet zur Herstellung enantiomerenreiner Verbindungen oder von Gemischen beliebiger Enantiomerenzusammensetzung.

## Beschreibung

Gegenstand der Erfindung ist ein Verfahren zur Herstellung von Acetalen und Ketalen von 3-Amino-5-(hydroxymethyl)cyclopentan-1,2-diolen, sowie deren Derivaten und Salzen organischer Säuren wie beispielsweise 2,3-Isopropylidendioxy-4-(methoxymethyl)cyclopentan-1-amin-Oxalat, ausgehend von 2-Acetyl-2-azabicyclo[2.2.1]hept-5-en-3-on der Formel

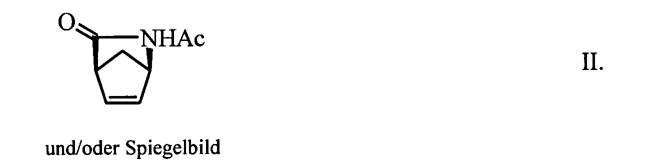

Unter enantiomerenreinen Verbindungen werden im Folgenden Enantiomerengemische mit einem Enantiomerenüberschuss (ee) von mindestens 90% verstanden.

Unter C₁₋₆-Alkyl wird im Folgenden eine lineare oder verzweigte aliphatische Alkylgruppe mit 1 bis 6 Kohlenstoffatomen wie beispielsweise Methyl, Ethyl, Propyl, Isopropyl, *n*-Butyl, Isobutyl, *sec*-Butyl, *tert*-Butyl, Pentyl, Hexyl verstanden.

Unter C₃₋₈-Cycloalkyl wird im Folgenden eine alicyclischer Alkylgruppe mit 3 bis 8 Kohlenstoffatomen wie beispielsweise Cyclopropyl, Cyclobutyl, Cyclopentyl, Cyclohexyl, Cycloheptyl oder Cyclooctyl verstanden.

Unter alkoholischer Lösung oder Suspension wird im Folgenden eine Lösung oder Suspension mindestens einer organischen Verbindung in einem beliebigen Gemisch aus Ethanol, Methanol, Propanol, Isopropylalkohol, *n*-Butanol, Isobutanol und/oder *tert*-Butanol, gegebenenfalls in Mischung mit Wasser und/oder weiteren Lösungsmitteln, Lösungsvermittlern oder weiteren Hilfsstoffen, verstanden.

Das Verfahren ist, abhängig von der Zusammensetzung des Eduktes, gleichermassen geeignet zur Herstellung von enantiomerenreinen Verbindungen oder von Gemischen beliebiger Enantiomerenzusammensetzung. Das jeweils nicht dargestellte Enantiomere soll in den Strukturformeldarstellungen durch die Ergänzung "und/oder Spiegelbild" sowie "oder Spiegelbild" mit umfasst sein.

Substituierte Cyclopentylamine, wie beispielsweise [1*R*,2*S*,3*R*,4*R*]-2,3-Isopropylidendioxy-4-(methoxymethyl)cyclopentan-1-amin-Hydrochlorid, sind Zwischenprodukte zur Herstellung von pharmazeutisch wirksamen Adenosinderivaten, die unter anderem zur Behandlung von koronaren Durchblutungsstörungen, Herzinsuffizienz und Bluthochdruck eingesetzt werden können (US-A-5364862; WO-A-95/28160; WO-A-97/11320 und WO-A-99/22932).

In einem bekannten Verfahren zur Herstellung von [1*R*,2*S*,3*R*,4*R*]-2,3-Isopropylidendioxy-4-(methoxymethyl)cyclopentan-1-amin-Hydrochlorid (WO-A-98/01426 und WO-A-00/23447) wird (-)-5,6-Isopropylidendioxy-2-azabicyclo[2.2.1]heptan-3-on unter Verwendung der sehr teuren und hydrolyseempfindlichen Verbindung Di-*tert*-butyldicarbonat zum entsprechend geschützten *N*-BOC-Derivat umgesetzt. Die Synthese des Eduktes (Verbindung "vi"), ausgehend von 5,6-Dihydroxy-2-azabicyclo[2.2.1]heptan-3-on ist in WO-A-95/28160 offenbart.
Nach erfolgter Lactamspaltung, gegebenenfalls gefolgt von einer Methylierung der neu entstandenen Hydroxylgruppe, wird die BOC-Schutzgruppe unter Verwendung von HCl-Gas abgespalten.
Bei diesem Verfahren werden Hydrochloride erhalten, die jedoch weder bezüglich Reinheit, Ausbeute oder chemisch-physikalischer Eigenschaften spezifiziert werden. Die gewonnenen Substanzen werden ohne weitere Aufarbeitung direkt in die jeweils nächsten Stufen eingesetzt.

Das im Stand der Technik offenbarte Verfahren zur Herstellung von [1*R*,2*S*,3*R*,4*R*]-2,3-Isopropylidendioxy-4-(methoxymethyl)cyclopentan-1-amin-Hydrochlorid weist Nachteile auf, die eine grosstechnische Umsetzung des Verfahrens verhindern. Aufgabe der vorliegenden Erfindung war es daher, ein grosstechnisch durchführbares und wirtschaftliches Verfahren zur Herstellung von Acetalen und Ketalen von 3-Amino-5-(hydroxymethyl)cyclopentan-1,2-diolen, sowie deren Derivaten und Salzen organischer Säuren zur Verfügung zu stellen.

Diese Aufgabe wurde in dem Verfahren gemäss Anspruch 1 gelöst, ausgehend von 2-Acetyl-2-azabicyclo[2.2.1]hept-5-en-3-on.

Nach dem erfindungsgemässen Verfahren können unter anderem enantiomerenreine Oxalate des [1*R*,2*S*,3*R*,4*R*]-2,3-Isopropylidendioxy-4-(hydroxymethyl)cyclopentan-1-amins und des [1*R*,2*S*,3*R*,4*R*]-2,3-Isopropylidendioxy-4-(methoxymethyl)cyclopentan-1-amins hergestellt werden.

Das erfindungsgemässe Verfahren kann mit (-)-2-Acetyl-2-azabicyclo[2.2.1]hept-5-en-3-on oder (+)-2-Acetyl-2-azabicyclo[2.2.1]hept-5-en-3-on, sowie einem beliebigen Gemisch davon durchgeführt werden.

Die Verbindung 2-Acetyl-2-azabicyclo[2.2.1]hept-5-en-3-on als Enantiomerengemisch, sowie die enantiomerenreine Form (-)-2-Acetyl-2-azabicyclo[2.2.1]hept-5-en-3-on, sind in WO-A-00/03032 offenbart.

Durch die Verwendung von acetylierten Lactamen kommt das Verfahren mit konventioneller und preiswerter Schutzgruppentechnik aus. Besonders gegenüber der, im Stand der Technik verwendeten, BOC-Schutzgruppe zeichnet sich die Verwendung der Acetylschutzgruppe im erfindungsgemässen Verfahren durch einfachere Handhabung aus.

Weiterhin lässt sich die Acetylgruppe leicht in einem alkalischen Hydrolyseschritt wieder entfernen, während das Verfahren aus WO-A-98/01426, für die Abspaltung der BOC-Schutzgruppe auf die Verwendung von HCl-Gas angewiesen ist. Gegenüber dem Verfahren aus WO-A-98/01426, bei dem in erheblichen Mengen NaCl-Abfall anfällt und das einen hohen Aufwand zum Korrosionsschutz von Anlagen und Leitungen erfordert, stellt das erfindungsgemässe Verfahren einen wesentlichen Fortschritt dar.

Es wurde festgestellt, dass sich nach dem erfindungsgemässen Verfahren hergestellte Acetale und Ketale von Cyclopentylaminen der Formel 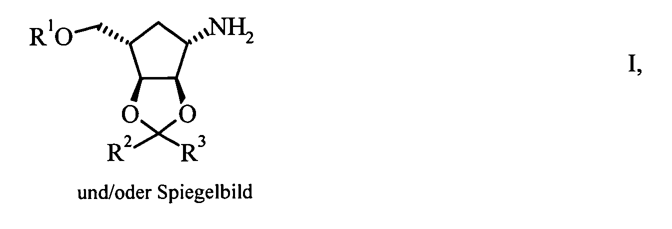 vorteilhaft als Oxalate fällen lassen, welche gegenüber den bereits bekannten Hydrochloriden wesentliche Vorteile in der Handhabung (Filtration, Zentrifugation) aufweisen.

Die nach dem erfindungsgemässen Verfahren herstellbaren Salze, wie beispielsweise [1*R*,2*S*,3*R*,4*R*]-2,3-Isopropylidendioxy-4-(methoxymethyl)cyclopentan-1-amin-Oxalat, können prinzipiell durch Anionenaustausch und erneute Fällung aus den entsprechenden Hydrochloriden erhalten werden. Sie sind jedoch nicht als direktes Hydrolyseprodukt aus der in WO-A 00/23447 genannten *N*-BOC-Verbindung erhältlich, da die BOC-Schutzgruppe zuerst mit einer starken Säure abgespalten werden muss. Weiterhin ist die Umfällung eines HCl-sauren Salzes mit einer organischen Säure nicht begünstigt.

Beschrieben wird ein Verfahren zur Herstellung von Verbindungen der Formel 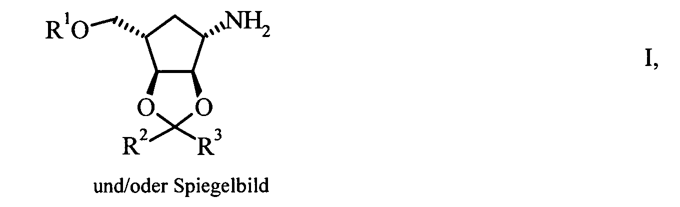 worin R¹ Wasserstoff, C₁₋₆-Alkyl, C₃₋₈-Cycloalkyl oder Benzyl sowie R² und R³ eine Kombination aus i) jeweils unabhängig voneinander Methyl und Ethyl ii) Wasserstoff und C₁₋₆-Alkyl oder Phenyl oder iii) eine Gruppe der Formel -(CH₂)ₙ- mit n = 4 bis 6 bedeuten, und die als freie Amine oder als Salze zwei- oder dreibasiger organischer Säuren vorliegen.
a) Hierzu wird in einem ersten Reaktionsschritt ein 2-Acetyl-2-azabicyclo[2.2.1]hept-5-en-3-on der Formel 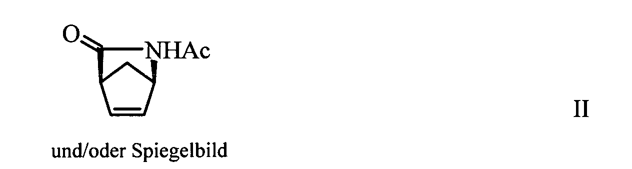 durch *cis*-Hydroxylierung der Doppelbindung in ein 2-Acetyl-5,6-dihydroxy-2-azabicyclo[2.2.1]heptan-3-on der Formel 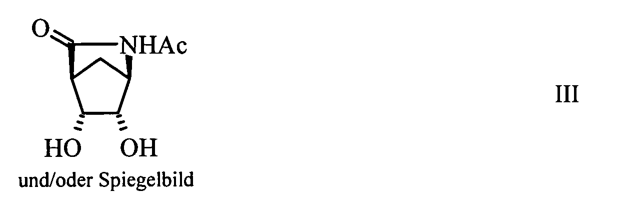 übergeführt.
b) In einem zweiten Reaktionsschritt wird eine Verbindung der Formel III durch Reaktion mit einem Keton oder einem Aldehyd der Formel R²-CO-R³ oder durch Reaktion mit 2,2-Dimethoxypropan oder 2,2-Dimethoxybutan, in ein Ketal oder Acetal der Formel 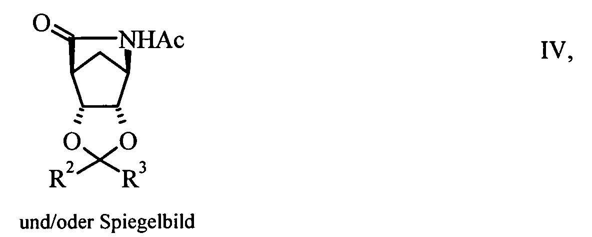 worin R² und R³ die genannten Bedeutungen haben, übergeführt.
c) Im folgenden Reaktionsschritt wird eine Verbindung der Formel IV durch reduzierende Ringöffnung in eine Verbindung der Formel 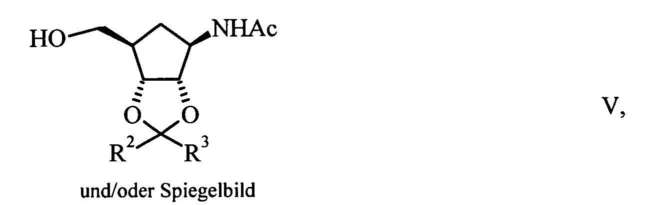 wobei R² sowie R³ die genannten Bedeutungen haben, übergeführt.
d) In einem gegebenenfalls folgenden Reaktionsschritt wird eine Verbindung der Formel V, oder gegebenenfalls ein Alkoxid davon, wobei R² und R³ die genannten Bedeutungen haben, durch Reaktion mit einem Alkylierungsmittel wie beispielsweise Dimethylsulfat (DMS), Benzylchlorid oder einem Halogenid der Formel R¹-X, worin R¹ die genannte Bedeutung ausser Wasserstoff hat und X = Brom oder Iod bedeutet, in eine Verbindung der Formel 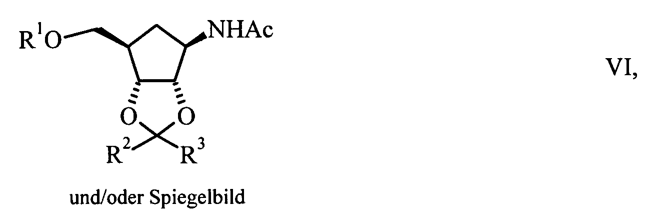 wobei R² sowie R³ die genannten Bedeutungen haben, übergeführt.
e) In einem weiteren Reaktionsschritt wird eine der in den Reaktionsschritten c) und/oder d) erhaltenen Verbindungen durch alkalische Hydrolyse in eine Verbindung der Formel 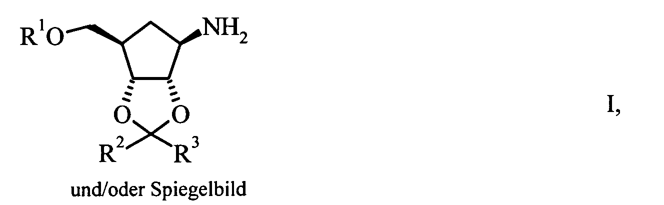 worin R¹, R² und R³ die genannten Bedeutungen haben, übergeführt.
f) In einem gegebenenfalls folgenden Reaktionsschritt werden Verbindungen der Formel I, mit einer zwei- oder dreibasigen organischen Säure zu den entsprechenden Salzen umgesetzt, worin R¹, R² und R³ die genannten Bedeutungen haben.

Zur Umsatzsteigerung und Einsparung des Oxidationsmittels vor der *cis*-Hydroxylierung werden störende Verbindungen aus der Synthese des 2-Acetyl-2-azabicyclo[2.2.1]hept-5-en-3-ons, wie beispielsweise 4-Dimethylaminopyridin (DMAP), vorteilhaft durch saure Extraktion in einem organischen Lösungsmittel gegen verdünnte, wässrige Säuren wie beispielsweise HCl oder H₂SO₄ entfernt.

In einer bevorzugten Ausführungsform wird die *cis*-Hydroxylierung der Doppelbindung in Reaktionsschritt a) unter Verwendung eines anorganischen Oxidationsmittels wie beispielsweise Osmiumtetraoxid, Kaliumosmat oder Kaliumpermanganat durchgeführt.

Wegen der Toxizität und Flüchtigkeit von Osmiumtetraoxid wird die Hydroxylierung in einer weiteren bevorzugten Ausführungsform unter Verwendung einer 2 bis 10%-igen wässrigen Lösung von Osmiumtetraoxid, oder mit auf einem organischen oder anorganischen Träger fixierten Osmiumtetraoxid durchgeführt.
In einer besonders bevorzugten Ausführungsform wird Osmiumtetraoxid in einer Menge von 0,1 bis 2 Mol-% bezüglich 2-Acetyl-2-azabicyclo[2.2.1]hept-5-en-3-on, vorzugsweise in einer Menge von 0,2 bis 0,9 Mol-%, durchgeführt.

Das Osmiumtetraoxid kann vorteilhaft während der Reaktion in Gegenwart von mindestens einem organischen *N*-Oxid wie beispielsweise 4-Methylmorpholin-4-oxid, und/oder einem sekundären oder tertiären Amin und mindestens einem anorganischen Oxidationsmittel wie beispielsweise Wasserstoffperoxid regeneriert werden.
Als Co-Oxidans zur Regenerierung von Osmiumtetraoxid während der Reaktion, eignen sich sterisch anspruchsvolle *N*-Oxide wie 4-Methylmorpholin-4-oxid, Di- und Trialkylamin-*N*-oxide wie Trimethylamin-*N*-oxid, oder Gemische der genannten Amine mit organischen oder anorganischen Oxidationsmitteln wie beispielsweise *tert*-Butylhydroperoxid, Magnesiummonoperoxyphthalat, 3-Chlorperbenzoesäure, Wasserstoffperoxid, Natrium- und/oder Kaliumperchlorat, -periodat oder -permanganat. Besonders bevorzugt ist die Verwendung von *N*-Oxiden und Gemischen aus den genannten Aminen mit Wasserstoffperoxid.

In einem besonders bevorzugten Verfahren wird enantiomerenreines (-)-2-Acetyl-2-azabicyclo[2.2.1]hept-5-en-3-on durch *cis*-Hydroxylierung mit Osmiumtetraoxid in [1*R*,4*S*,5*R*,6*S*]-2-Acetyl-5,6-dihydroxy-2-azabicyclo[2.2.1]heptan-3-on der Formel 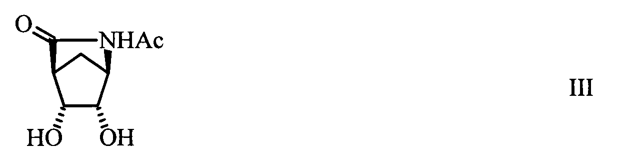 übergeführt.

In einer bevorzugten Verfahrensvariante wird die Acetal- bzw. Ketalbildung in Reaktionsschritt b) unter Säurekatalyse ausgeführt.

In einer besonders bevorzugten Verfahrensvariante wird für die Säurekatalyse Schwefelsäure und/oder p-Toluolsulfonsäure verwendet.

In einem bevorzugten Verfahren wird 2-Acetyl-5,6-dihydroxy-2-azabicyclo[2.2.1]heptan-3-on der Formel III durch Reaktion mit Aceton oder 2,2-Dimethoxypropan in 8-Acetyl-4,4-dimethyl-3,5-dioxa-8-azatricyclo[5.2.1.0^{2,6}]decan-9-on der Formel 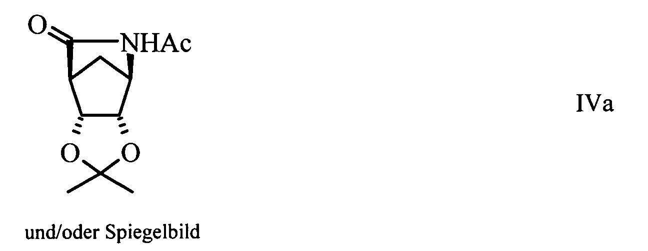 übergeführt.

In einer besonders bevorzugten Verfahrensvariante wird [1*R*,4*S*,5*R*,6*S*]-2-Acetyl-5,6-dihydroxy-2-azabicyclo[2.2.1]heptan-3-on durch Reaktion mit Aceton oder 2,2-Dimethoxypropan in (1*S*,2*R*,6*S*,7*R*)-8-Acetyl-4,4-dimethyl-3,5-dioxa-8-azatricyclo[5.2.1.0^{2,6}]decan-9-on der Formel 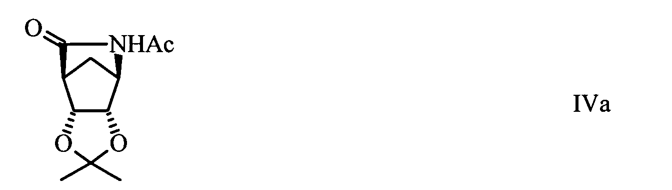 übergeführt.

In einem bevorzugten Verfahren wird die durch reduzierende Ringöffnung in Reaktionsschritt c) mit einem komplexen Metallhydrid wie beispielsweise LiBH₄, NaBH₄, NaAlH₂(OCH₂CH₂OCH₃)₂ oder LiAlH₄, bevorzugt mit NaBH₄, durchgeführt.

In einem weiteren bevorzugten Verfahren wird (1*S*,2*R*,6*S*,7*R*)-8-Acetyl-4,4-dimethyl-3,5-dioxa-8-azatricyclo[5.2.1.0^{2,6}]decan-9-on, durch Reaktion mit einem komplexen Metallhydrid in [1*R*,2*S*,3*R*,4*R*]-*N*-[2,3-Isopropylidendioxy-4-(hydroxymethyl)cyopentan-1-yl]essigsäureamid der Formel 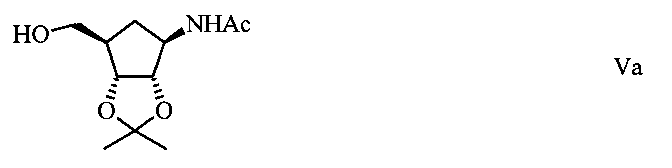 übergeführt.

In einer bevorzugten Verfahrensvariante wird in Reaktionsschritt d) die Alkylierung mit DMS in Aceton, in Gegenwart einer starken Base, wie beispielsweise NaOH und/oder KOH, durchgeführt, besonders bevorzugt mit einem Wassergehalt von unter 1 %, bezogen auf das Lösungsmittel.

In einer weiteren bevorzugten Verfahrensvariante wird die Alkylierung mit einem C₁₋₆-Alkyloder C₃₋₈-halogenid in Gegenwart eines Co-Katalysators, wie beispielsweise Ag(OH), durchgeführt.

In einem besonders bevorzugten Verfahren wird [1*R*,2*S*,3*R*,4*R*]-*N*-[2,3-Isopropylidendioxy-4-(hydroxymethyl)cyclopentan-1-yl]essigsäureamid durch Reaktion mit DMS oder Methyliodid in [1*R*,2*S*,3*R*,4*R*]-*N*-[2,3-Isopropylidendioxy-4-(methoxymethyl)cyclopentan-1-yl]essigsäureamid 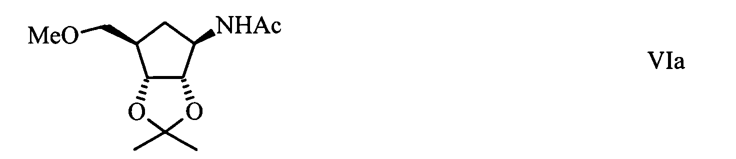 übergeführt.

In einer bevorzugten Verfahrensvariante wird die alkalische Hydrolyse in Reaktionsschritt e) mit mindestens einem Alkali- oder Erdalkalihydroxid, ausgewählt aus der Gruppe bestehend aus LiOH, NaOH, KOH, Mg(OH)₂, Ca(OH)₂ und Ba(OH)₂, in wässriger und/oder alkoholischer Lösung oder Suspension durchgeführt.

In einer bevorzugten Verfahrensvariante wird die alkalische Hydrolyse bei einem Druck von 1 bis 10 bar, besonders bevorzugt von 1 bis 2 bar, sowie bei Temperaturen von 50 bis 150 °C, besonders bevorzugt von 80 bis 100 °C durchgeführt werden.

In einer besonders bevorzugten Verfahrensvariante wird die alkalische Hydrolyse mit NaOH und/oder KOH in methanolischer und/oder ethanolischer Lösung, bei einem Druck von 1 bis 2 bar und einer Temperatur von 80 bis 100 °C durchgeführt.

In einem bevorzugten Verfahren wird durch alkalische Hydrolyse mit NaOH und/oder KOH in methanolischer und/oder ethanolischer Lösung, vorzugsweise unter einem Druck von 1 bis 2 bar und Temperaturen von 80 bis 100 °C,
i) die Verbindung [1*R*,2*S*,3*R*,4*R*]-*N*-[2,3-Isopropylidendioxy-4-(hydroxymethyl)cyclopentan-1-yl]essigsäureamid in die Verbindung [1*R*,2*S*,3*R*,4*R*]-2,3-Isopropylidendioxy-4-(hydroxymethyl)cyclopentan-1-amin bzw. 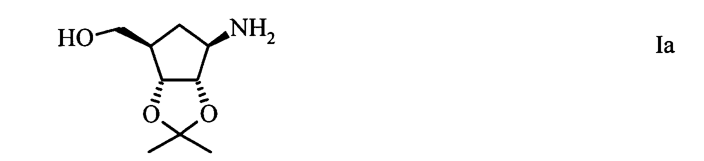
ii) die Verbindung [1*R*,2*S*,3*R*,4*R*]-*N*-[2,3-Isopropylidendioxy-4-(methoxymethyl)-cyclopentanl-yl]essigsäureamid in die Verbindung [1*R*,2*S*,3*R*,4*R*]-2,3-Isopropylidendioxy-4-(methoxymethyl)cyclopentan-1-amin 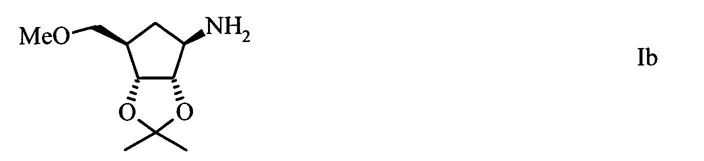 übergeführt.

In einer bevorzugten Verfahrensvariante wird die Salzbildung in Reaktionsschritt f) mit einer organischen Säure, ausgewählt aus der Gruppe bestehend aus Oxalsäure, (+)-, (-)- oder meso-Weinsäure, (+)- oder (-)-Äpfelsäure, Tartronsäure, Mesoxalsäure und Oxalessigsäure, sowie den jeweiligen Hydraten durchgeführt.

In einer besonders bevorzugten Verfahrensvariante wird die Salzbildung mit Oxalsäure, (+)-, (-)- oder meso-Weinsäure, (+)- oder (-)-Äpfelsäure durchgeführt.

In einer weiteren besonders bevorzugten Verfahrensvariante werden die Verbindungen [1*R*,2*S*,3*R*,4*R*]-2,3-Isopropylidendioxy-4-(hydroxymethyl)cyclopentan-1-amin oder [1*R*,2*S*,3*R*,4*R*]-2,3-Isopropylidendioxy-4-(methoxymethyl)cyclopentan-1-amin in die entsprechenden Oxalate übergeführt.

Von der Erfindung mit umfasst sind Verbindungen der Formel

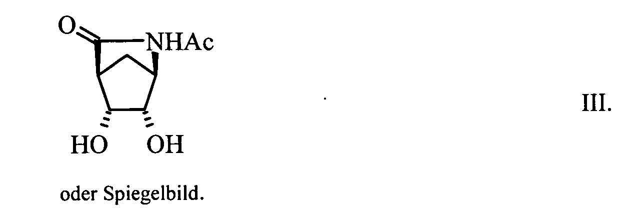

Ebenfalls von der Erfindung mit umfasst sind Verbindungen der Formel 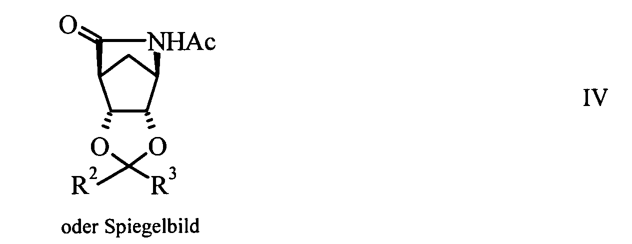 worin R² und R³ die oben genannten Bedeutungen haben.

Weiterhin sind von der Erfindung mit umfasst Verbindungen der Formel 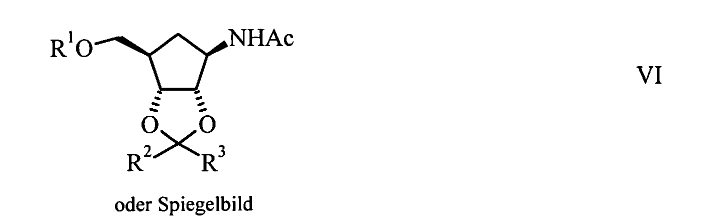 worin R¹ die genannte Bedeutung einschliesslich Wasserstoff hat und R² und R³ die oben genannten Bedeutungen haben.

Ausserdem sind von der Erfindung mit umfasst Salze zwei- und dreibasiger organischer Säuren von Verbindungen der Formel 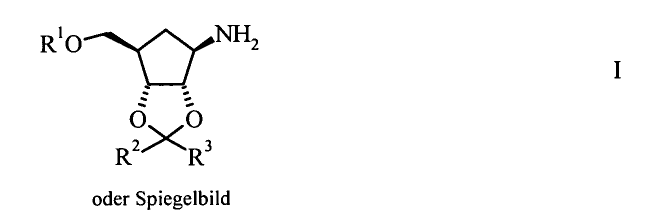 worin R¹, R² und R³ die oben genannten Bedeutungen haben.

### Beispiele

Obwohl die vorliegende Erfindung durch die angeführten erfindungsgemässen Beispiele 1 bis 22 vollständig offenbart wird, können aufgrund der beanspruchten Variationen der Verfahrensparameter noch zahlreiche weitere erfindungsgemässe Beispiele durchgeführt werden. Beispiele, die durch Umsetzen dieser, innerhalb der in der Beschreibung und in den Ansprüchen definierten Variationen durchgeführt werden, sollen als erfindungsgemässe Beispiele gelten und in den Schutzbereich dieser Patentanmeldung fallen.

### Beispiel 1

### [1R,4S,5R,6S]-2-Acetyl-5,6-dihydroxy-2-azabicyclo[2.2.1]heptan-3-on (Formel III)

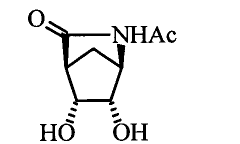

132,1 g (0,87 mol) (-)-2-Acetyl-2-azabicyclo[2.2.1]hept-5-en-3-on und 118,1 g (0,87 mol) 4-Methyl-morpholin-4-oxid · H₂O wurden in 750 mL Aceton, 224 mL entsalztem Wasser (E-Wasser) und 75 mL *tert*-Butanol vorgelegt. Die Reaktionslösung wurde auf 30 °C erwärmt. Eine Lösung von 2,0 g (7,9 mmol) Osmiumtetraoxid in 10 mL Aceton wurde während 15 Minuten zugetropft und das Reaktionsgemisch während 2 Stunden bei 30 °C gerührt. Anschliessend wurde die Reaktionslösung auf 10 °C abgekühlt und, zur Reduktion von überschüssigem 4-Methyl-morpholin-4-oxid und Osmiumtetraoxid, im Zeitraum von 1 Stunde mit 180,1 g (0,69 mol) 40%-iger Natriumhydrogensulfitlösung versetzt. Danach wurde das Reaktionsgemisch mit 35,1 g konz. Schwefelsäure auf pH 7 eingestellt. Die entstandene Suspension wurde filtriert und das Filtergut mit 20 mL Aceton nachgewaschen. Das Filtrat wurde unter Vakuum (40 bis 400 mbar) bei einer Temperatur von 40 °C auf 350 bis 400 mL eingeengt. Die Reaktionslösung wurde auf 20 °C gekühlt. Die wässrige Phase wurde mit 12,9 g konz. Schwefelsäure auf pH 2 eingestellt und nach der Abtrennung mit Ethylacetat (5 x 400 mL) extrahiert. Die vereinigten organischen Phasen wurden zur Trockene eingeengt. Der erhaltene Rückstand wurde über Nacht im Hochvakuum getrocknet.
Ausbeute: 81,6g (0,44 mol) [1*R*,4*S*,5*R*,6*S*]-2-Acetyl-5,6-dihydroxy-2-azabicyclo[2.2.1]-heptan-3-on, ca. 50% bezogen auf (-)-2-Acetyl-2-azabicyclo[2.2.1]hept-5-en-3-on
¹H-NMR (300 MHz) in CDCl₃: δ 4,89 (2H, s (breit)); 4,57 (1H, m); 4,17 (1H, m) 4,01 (1H, m); 2,83 (1H, m); 2,39 (3H, s); 2,14 (1H,m); 1,92 (1H, m).

### Beispiel 2

### [1S,2R,6S,7R]-8-Acetyl-4,4-dimethyl-3,5-dioxa-8-azatrieyclo[5.2.1.0^{2,6}]decan-9-on (Formel IVa = Formel IV mit R² = R³ = Methyl)

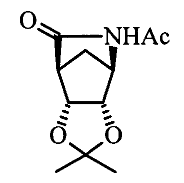

72,2 g (0,42 mol) [1*R*,4*S*,5*R*,6*S*]-2-Acetyl-5,6-dihydroxy-2-azabicyclo[2.2.1]heptan-3-on, 49,8 g (0,48 mmol) 2,2-Dimethoxypropan und 1,9 g (7,8 mmol) p-Toluolsulfonsäure wurden in 145 mL Ethanol vorgelegt. Die braune, klare Lösung wurde auf 50 °C erwärmt, 2 Stunden bei 50 °C gerührt, innerhalb von 30 Minuten auf 0 °C gekühlt und anschliessend mit 50 mL Ethanol versetzt. Die braune Suspension wurde 1 Stunde bei 0 °C gerührt. Die ausgefallenen Kristalle wurden abfiltriert und mit 25 mL Ethanol nachgewaschen. Das feuchte Produkt wurde bei 40 °C unter Vakuum getrocknet.
Ausbeute: 53,0g (0,24 mol) [1*S*,2*R*,6*S*,7*R*]-8-Acetyl-4,4-dimethyl-3,5-dioxa-8-azatricyclo[5.2.1.0^{2,6}]decan-9-on, ca. 56% bezogen auf [1*R*,4*S*,5*R*,6*S*]-2-Acetyl-5,6-dihydroxy-2-azabicyclo[2.2.1]heptan-3-on.
¹H-NMR (300 MHz) in CDCl₃: δ 4,74 (1H, m); 4,56 (1H, m); 4,43 (1H, m); 2,94 (1H, m); 2,41 (3H, s); 2,16 (1H, m); 1,95 (1H, m); 1,49 (3H, s); 1,34 (3H, s).

### Beispiel 3

### [1R,2S,3R,4R]-N-[2,3-Isopropylidendioxy-4-(hydroxymethyl)cyclopentan-yl]essigsäureamid (Formel Va = Formel V mit R² = R³ = Methyl)

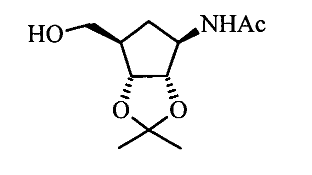

17,5 g (0,08 mol) [1*S*,2*R*,6*S*,7*R*]-8-Acetyl-4,4-dimethyl-3,5-dioxa-8-azatricyclo[5.2.1.0^{2,6}]-decan-9-on wurden in 250 mL Methanol vorgelegt und die klare farblose Lösung auf 0 °C gekühlt. Innerhalb von 1 Stunde wurden 6,6 g (0,17 mol) Natriumborhydrid portionsweise so zugegeben, dass die Temperatur unter 5 °C gehalten wurde.
Anschliessend wurde das Reaktionsgemisch innerhalb von 30 Minuten auf 20 °C erwärmt und 15 Stunden bei 20°C gerührt. Danach wurden 17,5 g Essigsäure in 10 Minuten zugetropft, das Reaktionsgemisch noch 10 Minuten weitergerührt und anschliessend zur Trockene eingedampft. Der Rückstand (44,8 g) wurde in 100 mL Ethylacetat aufgenommen und ca.
10 Minuten gerührt. Die ausgefallenen Kristalle wurden abfiltriert und mit Ethylacetat (2 x 20 mL) nachgewaschen. Das Filtrat wurde eingedampft und am Hochvakkum getrocknet. Das Rohprodukt (21,3 g) wurde mittels Säulenchromatographie an Kieselgel (Fliessmittel Ethylacetat/Methanol (5:1)) gereinigt.
Ausbeute 18,4 g (0,8 mmol) [1*R*,2*S*,3*R*,4*R*]-N-[2,3-Isopropylidendioxy-4-(hydroxymethyl)-cyclopentan-1-yl]essigsäureamid, ca. 100% bezogen auf [1*S*,2*R*,6*S*,7*R*]-8-Acetyl-4,4-dimethyl-3,5-dioxa-8-azatricyclo[5.2.1.0^{2,6}]decan-9-on.
¹H-NMR (300 MHz) in CDCl₃: δ 7,51 (1H, d); 4,60 (1H, m); 4.37 (1H, m); 4,34 (1H, m); 4,0 (1H, s, breit); 3,86 (1H, dd); 3,67 (1H, dd); 2,52 (1H, m); 2,33 (1H, m); 1,94 (3H, s); 1,49 (1H, m); 1,46 (3H, s); 1,28 (3H, s).

### Beispiel 4

### [1R,2S,3R,4R]-N-[2,3-Isopropylidendioxy-4-(methoxymethyl)yclopentan-1-yl]essigsäureamid (Formel IVa = Formel IV mit R¹ = R² = R³ = Methyl)

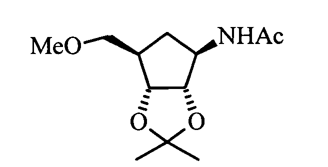

31,2 g (0,14 mol) [1*R*,2*S*,3*R*,4*R*]-N-[2,3-Isopropylidendioxy-4-(hydroxymethyl)cyclopentan-1-yl]essigsäureamid und 5,4 g Natronlauge (40%-ig) wurden in 250 mL Aceton vorgelegt und auf 50 °C erwärmt. Während 90 Minuten wurden parallel 20,2 g (0,16 mol) Dimethylsulfat und 5,7 g Natronlauge (40%-ig) bei 50 °C so zugetropft, dass der pH zwischen 11 und 13 gehalten wurde. Das Reaktionsgemisch wurde weitere 3 Stunden bei 50 °C gerührt und anschliessend im Vakuum auf ein Volumen von ca. 90 mL aufkonzentriert. Der Rückstand wurde mit 54 mL Wasser und 100 mL Ethylacetat versetzt und 30 Minuten gerührt. Die Phasen wurden getrennt und die wässrige Phase mit Ethylacetat (2 x 70 mL) extrahiert. Die vereinigten organischen Phasen wurden zur Trockene eingedampft.
Das Rohprodukt (31,5 g) wurde mittels Destillation gereinigt (Sdp. 120 bis 130 °C bei 0,15 mbar).
Ausbeute: 16,9 g (0,07 mmol) [1*R*,2*S*,3*R*,4*R*]-N-[2,3-Isopropylidendioxy-4-(methoxymethyl)-cyclopentan-1-yl]essigsäureamid, ca. 51% bezogen auf [1*R*,2*S*,3*R*,4*R*]-N-[2,3-Isopropylidendioxy-4-(hydroxymethyl)cyclopentan-1-yl]essigsäureamid.
¹H-NMR (300 MHz) in CDCl₃: δ 6,83 (1H, d); 4,52 (1H, m); 4,37 (1H, m); 4,34 (1H, m); 3,55 (1H, dd); 3,42 (1H, dd); 3,41 (3H, s); 2,53 (1H, m); 2,34 (1H, m); 1,93 (3H, s); 1,45 (3H, s); 1,42 (1H, m); 1,27 (3H, s).

### Beispiel 5

### [1R,2S,3R,4R]-2,3-Isopropylidendioxy-4-(methoxymethyl)cyclopentan-1-amin (Formel Ib = Formel I, R¹ = R² = R³ = Methyl)

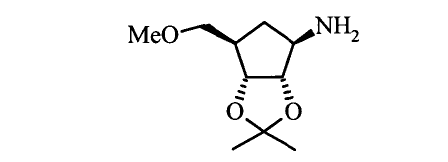

66,5 g (0,27 mol) [1*R*,2*S*,3*R*,4*R*]-N-[2,3-Isopropylidendioxy-4-(methoxymethyl)cyclopentan-1-yl]essigsäureamid wurden in 75 mL gesättigter Bariumhydroxidlösung vorgelegt und auf 100 °C erhitzt. Das Reaktionsgemisch wurde während 40 Stunden bei 100 °C unter Rückfluss gerührt, anschliessend auf 25 °C gekühlt und mit 32,5 mL E-Wasser und 160 mL Methylenchlorid versetzt. Das Gemisch wurde 30 Minuten bei 25 °C gerührt, anschliessend wurden die Phasen getrennt. Die wässrige Phase wurde nochmals mit 160 mL Methylenchlorid extrahiert und die vereinigten organischen Phasen zur Trockene eingeengt.
Ausbeute: 42,1 g (0,21 mol) [1*R*,2*S*,3*R*,4*R*]-2,3-Isopropylidendioxy-4-(methoxymethyl)-cyclopentan-1-amin, ca. 77% bezogen auf [1*R*,2*S*,3*R*,4*R*]-N-[2,3-Isopropylidendioxy-4-(methoxymethyl)cyclopentan-1-yl]essigsäureamid, ¹H-NMR (CDCl₃) entspricht.

### Beispiel 6

### [1R,2S,3R,4R]-2,3-Isopropylidendioxy-4-(methoxymethyl)cyclopentan-1-amin (Formel Ia = Formel I mit R¹ = R² = R³ = Methyl)

Eine Suspension von 2,3 g (9,6 mmol) [1*R*,2*S*,3*R*,4*R*]-N-[2,3-Isopropylidendioxy-4-(methoxymethyl)cyclopentan-1-yl]essigsäureamid und 1,5 mL Ba(OH)₂·H₂O (30%-ige Suspension in Wasser) in 1,2 mL Wasser wurde während 22 Stunden bei 100 °C unter Rückfluss gerührt. Die Suspension wurde auf 20 °C gekühlt, mit 50 mL Toluol versetzt und filtriert. Die Phasen wurden getrennt und die wässrige Phase wurde mit Toluol (2 x 50 mL) extrahiert. Die vereinigten organischen Phasen wurden bis zur Trockene eingeengt.
Ausbeute: 1,5g (7,5 mmol) [1*R*,2*S*,3*R*,4*R*]-2,3-Isopropylidendioxy-4-(methoxymethyl)-cyclopentan-1-amin als gelbe Flüssigkeit, ca. 78% bezogen auf [1*R*,2*S*,3*R*,4*R*]-N-[2,3-Isopropylidendioxy-4-(methoxymethyl)cyclopentan-1-yl]essigsäureamid.
¹H-NMR (300 MHz) in CDCl₃: δ 4,48 (1H, dd); 4,19 (1H, dd); 3,43 (2H, d); 3,37 (1H, m); 3,36 (3H, s); 2,30 (1H, m); 2,24 (1H, m); 1,47 (3H, s); 1,43 (2H, s, breit); 1,33 (1H, m); 1,29 (3H, s).

### Beispiel 7

### [1R,2S,3R,4R]-2,3-Isopropylidendioxy-4-(methoxymethyl)cyclopentan-1-amin-Oxalat (Salz der Verbindung der Formel I mit R¹ = R² = R³ = Methyl)

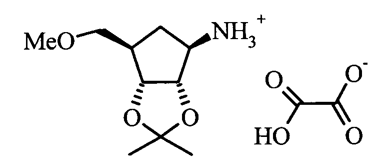

Zu einer Lösung von 42,1 g (0,21 mol) [1*R*,2*S*,3*R*,4*R*]-2,3-Isopropylidendioxy-4-(methoxymethyl)cyclopentan-1-amin in 107 mL Ethanol wurden bei 25 °C 17,0 g wasserfreie Oxalsäure (0,19 mol) portionsweise zugegeben. Die Reaktionslösung wurde während 30 Minuten bei 25 °C gerührt. Anschliessend wurden 430 mL Aceton und 55 mL Heptan nacheinander zugegeben, dann auf 0 °C abgekühlt und noch für weitere 60 Minuten bei 0 °C gerührt. Die ausgefallenen Kristalle wurden abfiltriert und mit 110 mL Heptan nachgewaschen. Der Filterrückstand wurde unter Vakuum bei 25 °C getrocknet.
Ausbeute: 42,7 g (0,15 mol) [1*R*,2*S*,3*R*,4*R*]-2,3-Isopropylidendioxy-4-(methoxymethyl)cyclopentan-1-amin-Oxalat, ca. 70% bezogen auf [1*R*,2*S*,3*R*,4*R*]-2,3-Isopropylidendioxy-4-(methoxymethyl)cyclopentan-1-amin.
¹H-NMR (300 MHz) in DMSO-d₆: δ 8,73 (4H, s, breit); 4,50 (1H, m); 4,40 (1H, m); 3,36 (3H, m); 3,27 (3H, s); 2,23 (2H, m); 1,57 (1H, m); 1,42 (3H, s); 1,23 (3H, s).

### Beispiel 8

### [1R,2S,3R,4R]-2,3-Isopropylidendioxy-4-(hydroxymethyl)cyclopentan-1-amin (Formel Ia = Formel I mit R¹ = H, R² = R³ = Methyl)

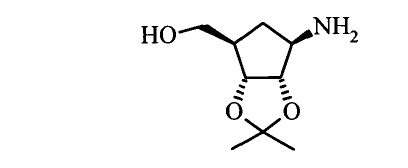

Eine Lösung von 3,5 g (15,3 mmol) [1*R*,2*S*,3*R*,4*R*]-N-[2,3-Isopropylidendioxy-4-(hydroxymethyl)cyclopentan-1-yl]essigsäureamid und 7,8 g 50%-ige Natronlauge wurden in einem Autoklav mit 25 mL Ethanol während 15,5 Stunden bei 100 °C und 2 bar erhitzt. Das Reaktionsgemisch wurde auf Raumtemperatur abgekühlt. Dann wurde die orange Suspension in 30 mL Ethanol gelöst und im Vakuum zur Trockene eingeengt. Der Rückstand wurde zweimal mit jeweils 10 mL Wasser versetzt und im Vakuum zur Trockene eingeengt. Anschliessend wurde der Rückstand mit Methyl-*tert*-butylether (MTBE) (2 × 10 mL) extrahiert und die vereinigten organischen Phasen im Vakuum zur Trockene eingedampft.
Ausbeute: 2,2 g (12,3 mmol) [1*R*,2*S*,3*R*,4*R*]-2,3-Isopropylidendioxy-4-(hydroxymethyl)cyclopentan-1-amin, ca. 80% bezogen auf [1*R*,2*S*,3*R*,4*R*]-N-[2,3-Isopropylidendioxy-4-(hydroxymethyl)cyclopentan-1-yl]essigsäureamid.
¹H-NMR (300 MHz) in CDCl₃: δ 4,78 (1H, d); 4,22 (1H, d); 3,72 (1H, dd); 3,55 (1H, dd); 3,53 (1H, m); 2,44 (2H, m); 1,44 (3H, s); 1,30 (1H, m); 1,29 (3H, s).

### Beispiel 10

### [1R,2S,3R,4R]-2,3-Isopropylidendioxy-4-(hydroxymethyl)cyclopentan-1-amin-Oxalat (Salz der Verbindung der Formel I mit R¹ = H, R² = R³ = Methyl)

Zu einer Lösung von 1,0 g (5,3 mmol) [1*R*,2*S*,3*R*,4*R*]-2,3-Isopropylidendioxy-4-(hydroxymethyl)cyclopentan-1-amin in 3 mL Ethanol wurden unter Rühren portionsweise 0,48 g (5,3 mmol) Oxalsäure und Ethanol (2 x 3 mL) zugegeben. Der weisse Niederschlag wurde abfiltriert und im Vakuum bei Raumtemperatur getrocknet.
Ausbeute: 1,0 g (3,7 mmol) [1*R*,2*S*,3*R*,4*R*]-2,3-Isopropylidendioxy-4-(hydroxymethyl)cyclopentan-1-amin-Oxalat, ca. 66% bezogen auf [1*R*,2*S*,3*R*,4*R*]-2,3-Isopropylidendioxy-4-(hydroxymethyl)cyclopentan-1-amin.
¹H-NMR (300 MHz) in DMSO-d₆: δ 6,95 (5H, s, breit); 4,46 (2H, m); 3,52 (1H, dd); 3,20 (2H, m); 2,26 (1H, m); 2,17 (1H, m); 1,57 (1H, m); 1,40 (3H, s); 1,23 (3H, s).

### Beispiel 11

### 8-Acetyl-4,4-dimethyl-3,5-dioxa-8-azatricyclo[5.2.1.0^{2,6}]decan-9-on Racemat (Formel IVa = Formel IV mit R² = R³ = Methyl)

Ein Gemisch von 72,2 g (0,39 mol) racemischem 2-Acetyl-2-azabicyclo[2.2.1]heptan-3-on, 49,8 g (0,47 mol) 2,2-Dimethoxypropan und 1,48 g (7,8 mmol) 4-Toluolsulfonsäure Monohydrat in 145 mL Ethanol wurde während 90 Min auf 50 °C erhitzt. Anschliessend wurde das Reaktionsgemisch während 30 Minuten auf 0 °C gekühlt und über 75 Minuten weitergerührt. Die ausgefallenen Kristalle wurden abfiltriert, mit 25 mL Ethanol gewaschen und bei 40 °C und 30 mbar getrocknet.
Ausbeute: 53,0 g (0,24 mol) 8-Acetyl-4,4-dimethyl-3,5-dioxa-8-azatricyclo[5.2.1.0^{2,6}]decan-9-on, ca. 60% bezogen auf 2-Acetyl-5,6-dihydroxy-2-azabicyclo[2.2.1]heptan-3-on, ¹H-NMR (CDCl₃) entspricht.

### Beispiel 12

### N-[2,3-Isopropylidendioxy-4-(hydroxymethyl)cyclopentan-1-yl]essigsäureamid Racemat (Formel Va = Formel V mit R² = R³ = Methyl)

Eine Lösung von 17,7 g (79 mmol) 8-Acetyl-4,4-dimethyl-3,5-dioxa-8-azatricyclo[5.2.1.0^{2,6}]-decan-9-on in 250 mL Methanol wurde unter N₂-Atmosphäre auf ca. 0 °C gekühlt. 6,6 g (175 mmol) Natriumborhydrid wurden portionsweise während 1 Stunde so zugegeben, dass die Temperatur unter 5 °C gehalten wurde. Das Reaktionsgemisch wurde über Nacht bei Raumtemperatur gerührt, mit einer Lösung von 17,5 g Essigsäure in 20 mL Methanol versetzt und im Vakuum bei 30 bis 35 °C zur Trockene gebracht. Der Rückstand wurde in 100 mL Ethanol aufgenommen und die weisse Suspension nach 10 Minuten Rühren filtriert. Der Filterrückstand wurde mit Ethylacetat (2 x 20 mL) gewaschen. Die vereinigten Filtrate wurden zur Trockene eingeengt und anschliessend im Hochvakuum getrocknet. (Ausbeute: 21,3 g Rohprodukt als gelbes viskoses Öl). 15,0 g des Rohproduktes wurden an Kieselgel 60 mit Ethylacetat/Methanol (5:1, v:v) chromatographiert.
Ausbeute: 13 g *N*-[2,3-Isopropylidendioxy-4-(hydroxymethyl)cyclopentan-1-yl]essigsäureamid (81 mmol) als gelbes viskoses Öl, ca. 100% bezogen auf 8-Acetyl-4,4-dimethyl-3,5-dioxa-8-azatricyclo[5.2.1.0^{2,6}]decan-9-on, ¹H-NMR (CDCl₃) entspricht.

### Beispiel 13

### N-[2,3-Isopropylidendioxy-4-(methoxymethyl)cyclopentan-1-yl]essigsäureamid Racemat (Formel VIa = Formel VI mit R¹ = R² = R³ = Methyl)

11,5 g (50 mmol) N-[2,3-Isopropylidendioxy-4-(hydroxymethyl)cyclopentan-1-yl]essigsäureamid wurden in 150 mL Aceton und 2,1 g 40%-iger Natronlauge auf ca. 50 °C erhitzt. Innerhalb von 90 Minuten wurden 7,7 g (61 mmol) Dimethylsulfat und 9,9 g 40%-ige Natronlauge parallel so zudosiert, dass die Temperatur bei 50 °C gehalten wurde. Das Reaktionsgemisch wurde während 3,5 Stunden bei 50 °C weitergerührt und dann im Vakuum auf ca. 30 mL konzentriert. Nach dem Abkühlen auf ca. 20 °C wurden 20 mL Wasser und 40 mL MTBE zugegeben. Die Phasen wurden getrennt und die wässrige Phase mit MTBE (2 x 25 mL) extrahiert. Einengen der vereinigten organischen Phasen zur Trockene ergab 11,5 g Rohprodukt.
Ausbeute: 11,5 g (47 mmol) *N*-[2,3-Isopropylidendioxy-4-(methoxymethyl)cyclopentan-1-yl]essigsäureamid als rot-braunes Öl (ca. 94% bezogen auf *N*-[2,3-Isopropylidendioxy-4-(hydroxymethyl)cyclopentan-1-yl]essigsäureamid), ¹H-NMR (CDCl₃) entspricht.

### Beispiel 14

### [1R,4S,5R,6S]-2-Acetyl-5,6-dihydroxy-2-azabicyclo[2.2.1]heptan-3-on (Formel III)

4,68 kg (-)-2-Acetyl-2-azabicyclo[2.2.1]hept-5-en-3-on (88%-ig) und 4,18 kg 4-Methylmorpholin-4-oxid Monohydrat wurden bei 20 °C in einem Gemisch aus 23,4 L Aceton, 7,0 L Wasser und 2,3 L *tert*-Butanol gelöst. Dann wurden 71 g Osmiumtetraoxid (0,9 Mol-% bezogen auf (-)-2-Acetyl-2-azabicyclo[2.2.1]hept-5-en-3-on) portionsweise so zugegeben, dass die Temperatur nicht über 35 °C angestiegen ist. Die Lösung wurde weitere 2 Stunden bei Raumtemperatur gerührt und dann bei 10 bis 15 °C im Verlauf von 1 Stunde mit 5,62 kg 40%-iger Natriumhydrogensulfit-Lösung versetzt. Anschliessend wurde das Reaktionsgemisch mit konz. Schwefelsäure auf pH = 7 eingestellt. Der Rückstand wurde abfiltriert und mit Aceton gewaschen. Die Mutterlauge und die Waschlauge wurden vereinigt. Dann wurden Aceton und *tert*-Butanol schonend bei 45 °C abdestilliert. Die braune Lösung wurde mit Schwefelsäure auf pH = 2 eingestellt und mit Ethylacetat (5 x 10 L) extrahiert. Die vereinigten organischen Phasen wurden zur Trockene eingeengt.
Ausbeute: 3,0 kg [1*R*,4*S*,5*R*,6*S*]-2-Acetyl-5,6-dihydroxy-2-azabicyclo[2.2.1]heptan-3-on, ca. 60% bezogen auf (-)-2-Acetyl-2-azabicyclo[2.2.1]hept-5-en-3-on

### Beispiel 15

### [1S,2R,6S,7R]-8-Acetyl-4,4-dimethyl-3,5-dioxa-8-azatricyclo[5.2.1.0^{2,6}]decan-9-on (Formel IVa = Formel IV mit R² = R³ = Methyl)

2,3 kg [1*R*,4*S*,5*R*,6*S*]-2-Acetyl-5,6-dihydroxy-2-azabicyclo[2.2.1]heptan-3-on wurden in 23 L Aceton gelöst und mit 23 g 4-Toluolsulfonsäure versetzt. Die Lösung wurde bei Raumtemperatur 24 Stunden gerührt. Die Reaktion wurde durch Zugabe von 230 g Natriumhydrogencarbonat abgebrochen, nachdem ein Acetonidgehalt von über 90% festgestellt wurde. Das Gemisch wurde für 1 Stunde bei Raumtemperatur gerührt, anschliessend filtriert und mit Aceton (2 x 800 mL) gewaschen. Das Filtrat wurde bei 30 mbar schonend auf ca. 10 Gew.-% eingeengt und dann mit 3,5 L *n*-Hexan versetzt. Nach dem Abkühlen auf Raumtemperatur wurde der Rückstand erneut filtriert, mit *n*-Hexan gewaschen und das Filtrat bei 40 °C und 30 mbar getrocknet.
Ausbeute: 1,9 kg [1*S*,2*R*,6*S*,7*R*]-8-Acetyl-4,4-dimethyl-3,5-dioxa-8-azatricyclo[5.2.1.0^{2,6}]-decan-9-on, 68% bezogen auf [1*R*,4*S*,5*R*,6*S*]-2-Acetyl-5,6-dihydroxy-2-azabicyclo[2.2.1]-heptan-3-on.

### Beispiel 16

### Vorreinigung: Extraktion von (-)-2-Acetyl-2-azabicyclo[2.2.1]hept-5-en-3-on (Formel II)

In einem 630 L Rührwerk (emaillierter Stahl) wurden 30 L Wasser vorgelegt und mit 8,35 kg Salzsäure (technisch, 32%-ig) versetzt. Anschliessend wurden 3,7 kg Natriumchlorid und 150 kg (-)-2-Acetyl-2-azabicyclo[2.2.1]hept-5-en-3-on (89%-ig) in den Reaktor gefüllt. Das Gemisch wurde bei Raumtemperatur mässig gerührt (Erfahrungsgemäss kann es bei allzu starker Rührgeschwindigkeit zur Emulsionsbildung kommen). Nach 30 Minuten wurde der Rührer abgestellt, und nach weiteren 30 Minuten konnten die Phasen sehr gut getrennt werden. Das erhaltene, gereinigte (-)-2-Acetyl-2-azabicyclo[2.2.1]hept-5-en-3-on wurde unmittelbar nach der Extraktion für die Herstellung von [1*R*,4*S*,5*R*,6*S*]-2-Acetyl-5,6-dihydroxy-2-azabicyclo[2.2.1]heptan-3-on eingesetzt.
Ausbeute: ca. 133 kg (-)-2-Acetyl-2-azabicyclo[2.2.1]hept-5-en-3-on (ca. 100%, ¹H-NMR (CDCl₃) entspricht.

### Beispiel 17

### [1R,4S,5R,6S]-2-Acetyl-5,6-dihydroxy-2-azabicyclo[2.2.1]heptan-3-on (Formel III)

In einem 2.500 L Rührwerk (Edelstahl) wurden 250 kg wässrige 4-Methyl-morpholin-4-oxid-Lösung (50%-ig), 132 L Wasser und 68 kg *tert*-Butanol vorgelegt und anschliessend mit 830 L Aceton versetzt. Zu dieser Mischung wurden bei Raumtemperatur 20 kg 4%-ige, wässrige Osmiumtetraoxid-Lösung gegeben. Danach wurden 150 kg (-)-2-Acetyl-2-azabicyclo[2.2.1]hept-5-en-3-on innerhalb von 1 Stunde so zudosiert, dass die Kesseltemperatur 30 bis 35 °C nicht überschritt und 1 Stunde weitergerührt. Bei einem Gehalte an restlichem (-)-2-Acetyl-2-azabicyclo[2.2.1]hept-5-en-3-on von unter 0,2% wurden innerhalb von 1 Stunde 58 kg wässrige Natriumhydrogensulfit-Lösung (40%-ig) bei 10 bis 50 °C zugegeben. Bei einer Temperatur von 10 bis 18 °C wurde die Reaktionslösung anschliessend mit Schwefelsäure (20%-ig) auf pH = 5 eingestellt.
Der Rückstand wurde abzentrifugiert und mit Aceton (2 x 20 L) gewaschen. Nach Vereinigung von Mutter- und Waschlauge wurde durch Abdestillieren von Aceton, *tert*-Butanol und Wasser bei 200 bis 300 mbar und einer Temperatur unter 45 °C aufkonzentriert.
Die Reaktionsmischung wurde auf 20 °C abgekühlt und mit Schwefelsäure (20%-ig) bei 12 bis 20 °C auf pH = 2 eingestellt. Anschliessend wurde mit Ethylacetat (4 x 670 L) extrahiert und die vereinigten organischen Phasen bei 30 bis 200 mbar und einer Temperatur von unter 45 °C bis zur Trockene eingeengt. Nach Ansteigen der Innentemperatur über 45 °C bei 30 bis 50 mbar wurde die Destillation abgebrochen und der Rückstand mit 620 L Methanol versetzt. Bei 200 mbar und einer Innentemperatur von 30 °C wurden Ethylacetat und Methanol bis zu einer Gesamtmenge von 400 L abdestilliert. Die Reaktionslösung wurde auf Raumtemperatur abgekühlt und dann direkt in die nächste Stufe zur Herstellung von [1*S*,2*R*,6*S*,7*R*]-8-Acetyl-4,4-dimethyl-3,5-dioxa-8-azatricyclo[5.2.1.0^{2,6}]decan-9-on eingesetzt.
Ausbeute ca. 59% bezogen auf [1*R*,4*S*,5*R*,6*S*]-2-Acetyl-5,6-dihydroxy-2-azabicyclo[2.2.1]-heptan-3-on, ¹H-NMR (CDCl₃) entspricht.

### Beispiel 18

### [1S,2R,6S,7R]-8-Acetyl-4,4-dimethyl-3,5-dioxa-8-azatricyclo[5.2.1.0^{2,6}]decan-9-on (Formel IVa = Formel IV mit R² = R³ = Methyl)

In einem 2.500 L Rührwerk (Edelstahl) wurden 21,0 kg Toluol-4-sulfonsäure Monohydrat vorgelegt und bei Raumtemperatur mit 1.240 kg [1*R*,4*S*,5*R*,6*S*]-2-Acetyl-5,6-dihydroxy-2-azabicyclo[2.2.1]heptan-3-on-Lösung (ca. 19%-ig) und 250 kg 2,2-Dimethoxypropan versetzt. Die Reaktionslösung wurde 1 Stunde bei 50 °C erwärmt, danach auf 35 °C abgekühlt und mit 1.200 L Methanol versetzt. Nach der Methanolzugabe wurde der [1*S*,2*R*,6*S*,7*R*]-8-Acetyl-4,4-dimethyl-3,5-dioxa-8-azatricyclo[5.2.1.0^{2,6}]decan-9-on-Gehalt bestimmt, die Lösung in Container abgefüllt und mit Methanol für die Herstellung von [1*R*,2*S*,3*R*,4*R*]-N-[2,3-Isopropylidendioxy-4-(hydroxymethyl)cyclopentan-1-yl]essigsäureamid verdünnt.
Ausbeute: ca. 195 kg [1*S*,2*R*,6*S*,7*R*]-8-Acetyl-4,4-dimethyl-3,5-dioxa-8-azatricyclo[5.2.1.0^{2,6}]decan-9-on (ca. 68% bezogen auf [1*R*,4*S*,5*R*,6*S*]-2-Acetyl-5,6-dihydroxy-2-azabicyclo[2.2.1]heptan-3-on, ¹H-NMR (CDCl₃) entspricht.

### Beispiel 19

### [1R,2S,3R,4R]-N-[2,3-Isopropylidendioxy-4-(hydroxymethyl)cyclopentan-1-yl]essigsäureamid (Formel Va = Formel V mit R² = R³ = Methyl), nicht isoliert

1323 kg einer [1*S*,2*R*,6*S*,7*R*]-8-Acetyl-4,4-dimethyl-3,5-dioxa-8-azatricyclo[5.2.1.0^{2,6}]decan-9-on-Lösung (ca. 5,3%) in Methanol wurden in ein 2.500 L Rührwerk (Edelstahl) eingefüllt und auf ca. 5 °C gekühlt. Im Verlauf von ca. 4 Stunden wurden 46 kg Natriumborhydrid in 12 Portionen von 3 bis 5 kg so zugegeben, dass im Rührwerk eine Innentemperatur von 10 °C nicht überschritten wurde. Nach beendeter Zugabe wurde das Reaktionsgemisch auf ca. 20 °C erwärmt, während 1 Stunde gerührt und anschliessend mit 53 L NaOH (30%-ig) versetzt. Das Methanol wurde wiederholt im Vakuum abdestilliert und die Blase entsprechend mit E-Wasser versetzt. Die wässrige Lösung wurde anschliessend mit 780 L Aceton versetzt. Nach der Phasentrennung und Entfernung der wässrigen Phase wurde die [1*R*,2*S*,3*R*,4*R*]-N-[2,3-Isopropylidendioxy-4-(hydroxymethyl)cyclopentan-1-yl]essigsäureamid-Lösung ohne Isolierung direkt weiter für die Herstellung von [1*R*,2*S*,3*R*,4*R*]-N-[2,3-Isopropylidendioxy-4-(methoxymethyl)cyclopentan-1-yl]essigsäureamid verwendet.

### Beispiel 20

### [1R,2S,3R,4R]-N-[2,3-Isopropylidendioxy-4-(methoxymethyl)cyclopentan-1-yl]essigsäureamid (Formel VIa = Formel VI mit R¹ = R² = R³ = Methyl), nicht isoliert

Zu 484 kg [1*R*,2*S*,3*R*,4*R*]-N-[2,3-Isopropylidendioxy-4-(hydroxymethyl)cyclopentan-1-yl]-essigsäureamid-Lösung in Aceton (ca. 15%-ig) wurden 70 kg Natriumhydroxid zugegeben und das Gemisch für 30 Minuten bei 25 °C gerührt. Nach einer Phasensetzzeit von 30 Minuten wurde die wässrige Phase entfernt. Nach Zugabe von weiteren 30 kg 30%-iger Natronlauge wurde das Gemisch auf 50 °C erhitzt. Danach wurden im Verlauf von 75 Minuten 123 kg Dimethylsulfat (DMS) und 30%-ige Natronlauge parallel so zudosiert, dass bei einer Innentemperatur von 50 °C ein pH-Wert von 11 bis 13 gehalten wurde. Die Reaktionslösung wurde nach 15 Minuten Nachrührzeit auf Raumtemperatur abgekühlt. Nach einer Phasensetzzeit von 30 Minuten wurde die wässrige Phase entfernt. Das Aceton wurde im Vakuum bis auf eine Restmenge von 500 L abdestilliert und die [1*R*,2*S*,3*R*,4*R*]-N-[2,3-Isopropylidendioxy-4-(methoxymethyl)cyclopentan-1-yl]essigsäureamid-Lösung nach Abkühlung auf 25 °C direkt weiter zu [1*R*,2*S*,3*R*,4*R*]-2,3-Isopropylidendioxy-4-(methoxymethyl)cyclopentan-1-amin umgesetzt.
Die Ausbeute wurde nicht bestimmt und die Lösung direkt zur Herstellung von [ 1*R*,2*S*,3*R*,4*R*]-2,3-Isopropylidendioxy-4-(methoxymethyl)cyclopentan-1-amin verwendet.
¹H-NMR (CDCl₃) entspricht.

### Beispiel 21

### [1R,2S,3R,4R]-2,3-Isopropylidendioxy-4-(methoxymethyl)cyclopentan-1-amin (Formel Ia = Formel I mit R¹ =R² = R³ = Methyl)

In einem 630 L Rührwerk (Edelstahl) wurden 460 kg [1*R*,2*S*,3*R*,4*R*]-N-[2,3-Isopropylidendioxy-4-(methoxymethyl)cyclopentan-1-yl]essigsäureamid-Lösung in Aceton (ca.13.6%-ig) vorgelegt. Bei 30 bis 300 mbar und einer Temperatur unter 45 °C wurde das Aceton bis zum Erreichen eines Acetongehaltes von unter 0, 1 % abdestilliert und das Reaktionsgemisch mit 400 L Ethanol versetzt. Anschliessend wurden bei Raumtemperatur 126 kg Natronlauge (50%-ig) zugegeben und die Hydrolyse wurde bei ca. 100 °C und 2 bar im Verlauf von 8 Stunden durchgeführt. Die Reaktionslösung wurde auf Raumtemperatur abgekühlt und Ethanol bei 100 mbar und einer Temperatur von unter 45 °C abdestilliert. Der Rückstand wurde mit 180 L Wasser versetzt und die restliche Ethanolmenge abdestilliert. Im Reaktor befanden sich nach der Destillation ca. 270 L Reaktionsgemisch, das mit 140 L Wasser versetzt und anschliessend mit MTBE (2 x 145 L) extrahiert wurde. Die vereinigten organischen Phasen wurden direkt zur Herstellung von [1*R*,2*S*,3*R*,4*R*]-2,3-Isopropylidendioxy-4-(methoxymethyl)cyclopentan-1-amin-Oxalat eingesetzt. Der Umsatz von [1*R*,2*S*,3*R*,4*R*]-N-[2,3-Isopropylidendioxy-4-(methoxymethyl)cyclopentan-1-yl]essigsäureamid in [1*R*,2*S*,3*R*,4*R*]-2,3-Isopropylidendioxy-4-(methoxymethyl)cyclopentan-1-amin war >96.5%. ¹H-NMR (CDCl₃) entspricht.

### Beispiel 22

### [1R,2S,3R,4R]-2,3-Isopropylidendioxy-4-(methoxymethyl)cyclopentan-1-amin-Oxalat (Salz der Verbindung der Formel I mit R¹ = R² = R³ = Methyl)

In einem 630 L Rührwerk (emaillierter Stahl) wurden zu einer Vorlage aus 23,3 kg Oxalsäure und 210 L Ethanol die in Beispiel 4.6 beschriebene [1*R*,2*S*,3*R*,4*R*]-2,3-Isopropylidendioxy-4-(methoxymethyl)cyclopentan-1-amin-Lösung im Verlauf von 30 Minuten zudosiert. Nach Zugabe von 60% wurde die Lösung durch Zugabe von wenig [1*R*,2*S*,3*R*,4*R*]-2,3-Isopropylidendioxy-4-(methoxymethyl)cyclopentan-1-amin-Oxalat angeimpft Das ausgefallene Produkt wurde mit MTBE/Ethanol (60 : 40) (2 × 120 L) gewaschen und abzentrifugiert. Ausbeute: 56 kg [1*R*,2*S*,3*R*,4*R*]-2,3-Isopropylidendioxy-4-(methoxymethyl)cyclopentan-1-amin-Oxalat (ca. 78% bezogen auf [1*R*,2*S*,3*R*,4*R*]-N-[2,3-Isopropylidendioxy-4-(methoxymethyl)cyclopentan-1-yl]essigsäureamid), ¹H-NMR (CDCl₃) entspricht.

## Patentansprüche

1. Verfahren zur Herstellung von Verbindungen der Formel, 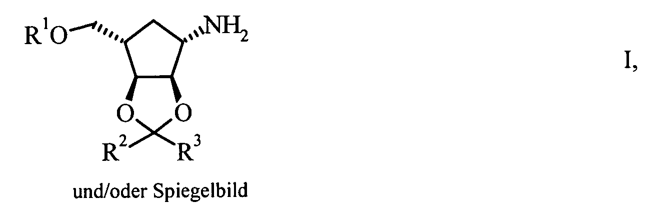 worin R¹ Wasserstoff, C₁₋₆-Alkyl, C₃₋₈-Cycloalkyl oder Benzyl sowie R² und R³ jeweils unabhängig voneinander eine Kombination aus Methyl und Ethyl, Wasserstoff und C₁₋₆-Alkyl, Wasserstoff und Phenyl, oder gemeinsam eine Gruppe der Formel -(CH₂)ₙ- mit n = 4 bis 6 bedeuten, und die als freie Amine oder als Salze organischer zwei- oder dreibasiger Säuren vorliegen, **dadurch gekennzeichnet, dass**
a) ein 2-Acetyl-2-azabicyclo[2.2.1]hept-5-en-3-on der Formel 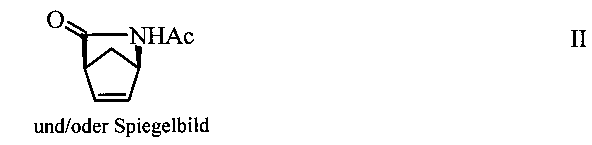 durch *cis*-Hydroxylierung der Doppelbindung in ein 2-Acetyl-5,6-dihydroxy-2-azabicyclo[2.2.1]heptan-3-on der Formel 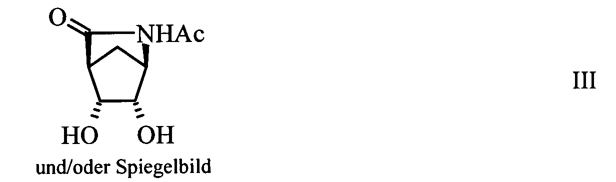 übergeführt wird, und dieses
b) durch Reaktion mit einem Keton oder einem Aldehyd der Formel R²-CO-R³, wobei R² und R³ die genannten Bedeutungen haben, oder durch Reaktion mit 2,2-Dimethoxypropan oder 2,2-Dimethyoxybutan, in ein Keton oder Acetal der Formel 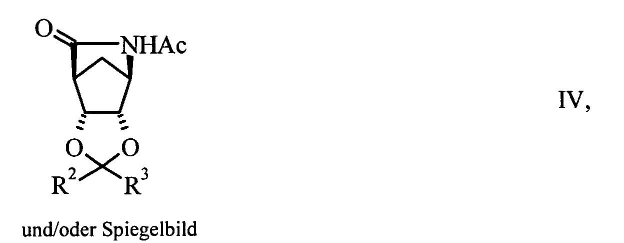 worin R² und R³ die genannten Bedeutungen haben, übergeführt wird und dieses
c) durch reduzierende Ringöffnung in eine Verbindung der Formel 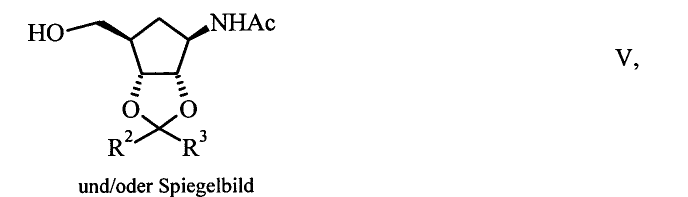 worin R² und R³ die genannten Bedeutungen haben, übergeführt wird und dieses gegebenenfalls,
d) durch Alkylierung mit Dimethylsulfat, Benzylchlorid oder einem Halogenid der Formel R¹-X, worin R¹ die genannte Bedeutung ausser Wasserstoff hat und X = Brom oder Jod bedeutet, in eine Verbindung der Formel 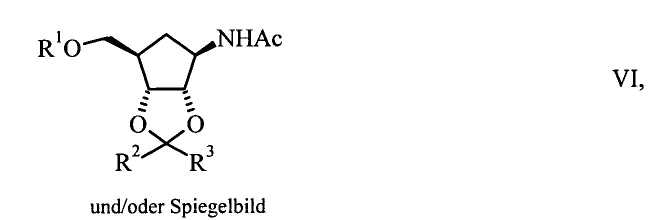 worin R² bzw. R³ die genannten Bedeutungen haben, übergeführt wird, und eine der in den Reaktionsschritten c) oder d) erhaltenen Verbindungen
e) durch alkalische Hydrolyse in eine Verbindung der Formel 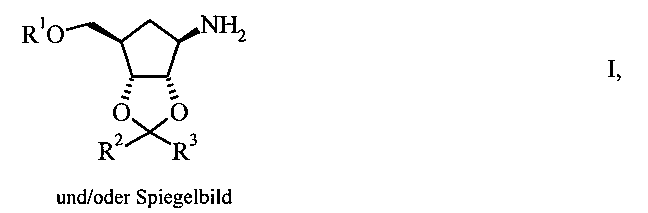 worin R¹, R² und R³ die genannten Bedeutungen haben, übergeführt wird, und dieses gegebenenfalls
f) durch Zugabe einer zwei- oder dreibasigen organischen Säure zu dem entsprechenden Salz umgesetzt wird, worin R¹, R² und R³ die genannten Bedeutungen haben.

2. Verfahren nach Anspruch 1, **dadurch gekennzeichnet, dass** die *cis*-Hydroxylierung der Doppelbindung in Reaktionsschritt a) unter Verwendung von Osmiumtetraoxid durchgeführt wird.

3. Verfahren nach Anspruch 2, **dadurch gekennzeichnet, dass** Osmiumtetraoxid in einer Menge von 0,1 bis 2,0 Mol-%, vorzugsweise in einer Menge von 0,2 bis 0,9 Mol-%, bezogen auf die Verbindungen der Formel II verwendet wird und dieses während der Reaktion regeneriert wird.

4. Verfahren nach Anspruch 3, **dadurch gekennzeichnet, dass** das Osmiumtetraoxid durch Zusatz eines sterisch anspruchsvollen *N*-Oxids oder eines Gemisches eines sterisch anspruchsvollen Amins mit Wasserstoffperoxid regeneriert wird.

5. Verfahren nach mindestens einem der Ansprüche 1 bis 4, **dadurch gekennzeichnet, dass** die Ketal- oder Acetalbildung in Reaktionsschritt b) unter Säurekatalyse durchgeführt wird.

6. Verfahren nach Anspruch 5, **dadurch gekennzeichnet, dass** für die Säurekatalyse Schwefelsäure und/oder p-Toluolsulfonsäure verwendet wird.

7. Verfahren nach mindestens einem der Ansprüche 1 bis 6, **dadurch gekennzeichnet, dass** die Ketal- oder Acetalbildung mit Aceton oder 2,2-Dimethoxypropan durchgeführt wird.

8. Verfahren nach mindestens einem der Ansprüche 1 bis 7, **dadurch gekennzeichnet, dass** die reduzierende Ringöffnung mit einem komplexen Metallhydrid, vorzugsweise mit NaBH₄ durchgeführt wird.

9. Verfahren nach mindestens einem der Ansprüche 1 bis 8, **dadurch gekennzeichnet, dass** die Alkylierung mit Dimethylsulfat durchgeführt wird.

10. Verfahren nach mindestens einem der Ansprüche 1 bis 8, **dadurch gekennzeichnet, dass** die Alkylierung mit Methyliodid durchgeführt wird.

11. Verfahren nach mindestens einem der Ansprüche 1 bis 10, **dadurch gekennzeichnet, dass** die alkalische Hydrolyse mit mindestens einem Alkali- oder Erdalkalihydroxid, ausgewählt aus der Gruppe bestehend aus LiOH, NaOH, KOH, Ca(OH)₂, Ba(OH)₂, in wässriger und/oder alkoholischer Lösung oder Suspension durchgeführt wird.

12. Verfahren nach Anspruch 11, **dadurch gekennzeichnet, dass** die alkalische Hydrolyse bei einem Druck von 1 bis 10 bar, besonders bevorzugt von 1 bis 2 bar, sowie bei Temperaturen von 50 bis 150 °C, besonders bevorzugt von 80 bis 100 °C durchgeführt wird.

13. Verfahren nach mindestens einem der Ansprüche 1 bis 12, **dadurch gekennzeichnet, dass** die organischen Säure ausgewählt wird aus der Gruppe bestehend aus Oxalsäure, (+)-, (-)- oder *meso*-Weinsäure, (+)- oder (-)-Äpfelsäure, Tartronsäure, Mesoxalsäure und Oxalessigsäure, sowie den jeweiligen Hydraten.

14. Verfahren nach Anspruch 13, **dadurch gekennzeichnet, dass** als organische Säure Oxalsäure und/oder deren Hydrat verwendet wird.

15. Verbindungen der Formel

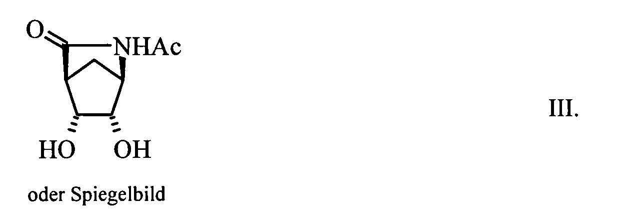

16. Verbindungen der Formel 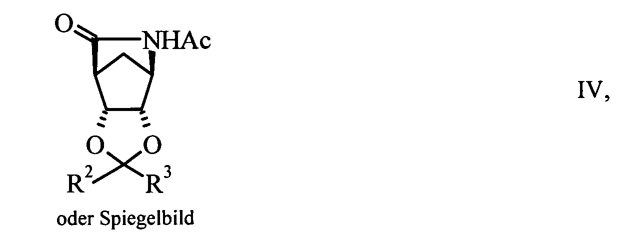 worin R² und R³ die in Anspruch 1 genannten Bedeutungen haben.

17. Verbindungen der Formel 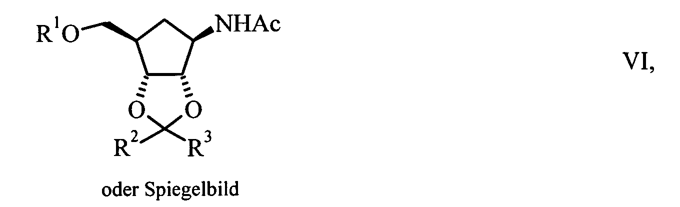 worin R¹, R² und R³ die in Anspruch 1 genannten Bedeutungen haben.

18. Salze zwei- und dreibasiger organischer Säuren von Verbindungen der Formel 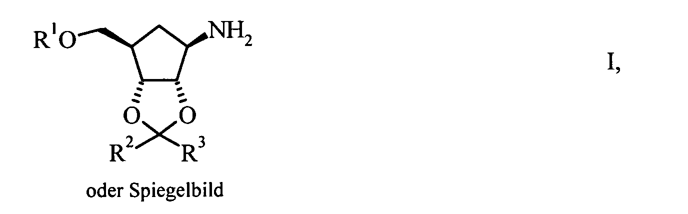 worin R¹, R² und R³ die in Anspruch 1 genannten Bedeutungen haben.
